# EUROPEAN PATENT APPLICATION

(11) **EP 4 585 176 A1**
(43) Date of publication of application: **16.07.2025**
(21) Application number: 23885137.2
(22) Date of filing: 06.11.2023
(51) Int. Cl.: A61B 18/12, A61B 18/00

(54) **ELECTROSURGICAL INSTRUMENT**

(30) Priority: 04.11.2022 CN 202211378660
(71) Applicant: Reach Surgical, Inc., Tianjin 300462 (CN)
(72) Inventor: WANG, Zhen, Tianjin 300462 (CN); YANG, Chao, Tianjin 300462 (CN); ZHANG, Xiaoqiang, Tianjin 300462 (CN); GE, Juan, Tianjin 300462 (CN); LI, Sandong, Tianjin 300462 (CN)
(74) Representative: Sun, Yiming
(86) International application number: PCT/CN2023/129961
(87) International publication number: WO 2024/094213

(57) **Abstract**

Disclosed is an electrosurgical instrument, relating to the field of medical instruments, and including: an end effector; and a handle assembly operably providing driving force and electrosurgical energy to the end effector, the handle assembly including a handle portion and a closure trigger pivotally connected to the handle portion. The handle portion has a first accommodating chamber, the closure trigger has a second accommodating chamber, and an activation switch capable of turning on or off activation of transmission of the electrosurgical energy is provided in the first accommodating chamber and/or the second accommodating chamber. When the closure trigger pivots towards the handle portion side to an activation position, the activation switch is triggered into an activated state to provide the electrosurgical energy to the end effector. The surgical instrument of the present invention shortens the operation time of closing and coagulating (cauterizing and sealing) tissue, and also avoids the problem of inadvertent operation on the activation switch.

## Description

The present application claims the priority of Chinese patent application No. 202211378660.4 filed with the China National Intellectual Property Administration on November 4, 2022, and entitled "Electrosurgical Instrument", which is incorporated herein by reference in its entirety.

### TECHNICAL FIELD

The present invention relates to the field of medical instruments, and in particular to an electrosurgical instrument.

### BACKGROUND

A high-frequency electrosurgical instrument is a device used for tissue cutting, which heats tissue using a high-frequency high-voltage current generated at electrode tips when in contact with body, so as to achieve tissue transection and coagulation, thereby achieving the purpose of cutting and hemostasis. A bipolar electrosurgical instrument is a high-frequency electrosurgical instrument having bipolar functionality, allowing high-frequency, high-voltage currents to flow between the two electrodes of the bipolar device, causing dehydration and shrinkage of vessel walls, coagulation of blood within the vessels, and fusion of the vessels with blood clots, thereby achieving tissue transection and coagulation. Bipolar electrosurgical instruments are widely used in various precision surgical procedures, such as neurosurgery, vascular surgery, microsurgery, and plastic surgery, due to its minimal thermal damage, controllable cutting speed, and adjustable coagulation depth.

A bipolar electrosurgical instrument typically includes a handle assembly, an elongated body assembly, and an end effector. The handle assembly is operatable connected to an electrosurgical generator adapted for outputting a high-frequency high-voltage power. The end effector includes a pair of grasping members used to clamp tissue to be cut. The grasping members are actuated by a driving mechanism provided within the handle assembly and a transmission mechanism provided in the handle assembly and the elongated body assembly. During use, a trigger on the handle assembly is operated to close the grasping members to hold the tissue to be cut. Subsequently, an activation button on the handle assembly is pressed so as to allow delivery of high-frequency energy. However, since tissue clamping and energy activation are separate operations, the multistep process provides surgical inefficiency and ergonomic discomfort. Moreover, for some of the bipolar instruments, the activation button is exposed on the handle assembly, increasing the risks of accidental activation.

### SUMMARY

It is provided in the present invention an electrosurgical instrument that is easy to operate with low safety risks.

It is provided in the present invention an electrosurgical instrument, comprising: an end effector; and a handle assembly operably providing driving force and electrosurgical energy to the end effector, the handle assembly comprising a handle portion and a closure trigger pivotally coupled to the handle portion, wherein a first accommodating chamber is arranged in the handle portion, a second accommodating chamber is arranged in the closure trigger, and an activation switch is arranged in the first accommodating chamber and/or the second accommodating chamber for enabling or disabling delivery of the electrosurgical energy, and when the closure trigger is operated to pivot toward the handle portion to an activation position, the activation switch is triggered into an activated state allowing the electrosurgical energy delivered to the end effector.

According to an embodiment, when the end effector is in an open state, at least a portion of the closure trigger is received in the first accommodating chamber of the handle portion.

According to an embodiment, when the closure trigger is pivoted toward the handle portion, the area of the portion of the closure trigger received within the first accommodating chamber increases; and as the closure trigger is pivoted away from the handle portion, the area of the portion of the closure trigger received within the first accommodating chamber decreases.

According to an embodiment, further comprising an electrical connection portion for being electrically connected to an electrosurgical generator for generating the electrosurgical energy, and a control circuit board electrically connected to the electrical connection portion; wherein an activation circuit is arranged on the control circuit board, and the activation switch is operably configured to enable or disable the activation circuit to control delivery of the electrosurgical energy to the end effector.

According to an embodiment, wherein the activation switch is a button switch, and a trigger assembly for triggering the activation switch is further provided in the first accommodating chamber and/or the second accommodating chamber, and when the closure trigger is pivoted to the activation position, the trigger assembly is biased from an initial state and acts on the activation switch to enable the activation switch.

According to an embodiment, wherein the activation switch is provided in the second accommodating chamber, and when the closure trigger is pivoted to the activation position, the activation switch is operably triggered into the activated state.

According to an embodiment, wherein the second accommodating chamber further comprises a trigger assembly having a trigger lever operable to be moved from an initial state so as to enable the activation switch, and the trigger lever is biased in the initial state through a biasing member engaged therewith.

According to an embodiment, wherein a mounting seat is provided in the second accommodating chamber, the trigger lever of the trigger assembly is slidably provided on the mounting seat, and the activation switch is operated to be turned on or off through sliding of the trigger lever on the mounting seat in a first direction, and the biasing member is provided on the mounting seat to provide the biasing force to the trigger lever.

According to an embodiment, wherein one end of the biasing member acts on the activation switch, the other end acts on the trigger lever of the trigger assembly, and the activation switch is maintained in the activated state during at least a portion of movement of the closure trigger pivoting from the activation position towards an open position.

According to an embodiment, wherein a mounting seat is provided in the second accommodating chamber, the trigger lever of the trigger assembly is pivotally provided on the mounting seat, and the activation switch is operated to be turned on or off as the trigger lever pivots on the mounting seat in a third direction, and the biasing member is provided on the mounting seat to provide the biasing force to the trigger lever.

According to an embodiment, wherein a contact portion for being engaged with the trigger assembly is provided in the first accommodating chamber, and when the closure trigger is pivoted to the activation position, the contact portion abuts one end of the trigger lever, so that the trigger lever overcomes biasing force of the biasing member and enables the activation switch.

According to an embodiment, wherein the activation switch and a control circuit board are mounted on the mounting seat.

According to an embodiment, wherein a trigger assembly is further provided in the second accommodating chamber, and is provided on a housing surface of the closure trigger for a user to hold, and when the closure trigger pivots to the activation position, the trigger assembly operably enables the activation switch.

According to an embodiment, wherein the closure trigger has a mounting port, a pressing side of the activation switch faces the mounting port, the trigger assembly comprises a sealing cover body mounted on the mounting port, the sealing cover body has an elastically deformable region opposite the activation switch, and the elastically deformable region of the sealing cover body is capable of being triggered and deforming in a direction close to the activation switch to a set position to turn on the activation switch.

According to an embodiment, wherein force for triggering pivoting the closure trigger is less than elastic deformation force for the sealing cover body.

According to an embodiment, wherein a trigger assembly is further provided in the first accommodating chamber, and the trigger assembly comprises a trigger lever slidably connected to a mounting seat, the trigger lever sliding on the mounting seat in a second direction to trigger the activation switch to be turned on or off; and a biasing member mounted on the mounting seat and acting on the trigger lever.

According to an embodiment, wherein the activation switch is a contact switch, and the activation switch comprises:
at least one first contact located in the first accommodating chamber and at least one second contact located in the second accommodating chamber, the first contact being electrically connected to the electrical connection portion, the first contact or the second contact being electrically connected to the control circuit board, and
when the closure trigger is operated to pivot to the activation position, the second contact abutting the first contact to enable the activation circuit.

According to an embodiment, wherein a guide structure is provided between the handle portion and the closure trigger, and the guide structure is used for guiding a swinging direction of the closure trigger.

According to an embodiment, wherein the handle assembly further comprises a first biasing member and a second biasing member, and the closure trigger is operated to pivot toward the handle portion to a closed position by overcoming elastic force of the second biasing member, and further to pivot toward the handle portion to the activation position by overcoming elastic force of the first biasing member and the second biasing member.

According to an embodiment, further comprising an elongated body assembly defining a longitudinal axis, and comprising an outer sheath, and a proximal end of the outer sheath extending to the inside of the handle portion.

According to an embodiment, wherein the closure trigger is operably coupled on a proximal portion of the outer sheath via a connection portion formed on an upper end portion of the closure trigger and pivotally mounted on the handle portion.

According to an embodiment, wherein the handle portion is provided with a stopper located at a proximal portion of the outer sheath, a proximal washer, a driving collar, and a snap ring are sequentially sleeved on the outer sheath located in the handle portion from the proximal end to a distal end, the snap ring and the proximal washer are separately fixedly connected to the outer sheath, the driving collar is slidable along the outer sheath, a proximal end of the driving collar is provided with a spring washer, the connection portion of the closure trigger is sleeved on the driving collar, two ends of the connection portion respectively abut the spring washer and the snap ring, the first biasing member is provided between the spring washer and the proximal washer, the second biasing member is provided between the proximal washer and the stopper, and an elastic coefficient of the first biasing member is greater than an elastic coefficient of the second biasing member.

According to an embodiment, wherein the handle assembly further comprises a cutting trigger pivotally connected to the handle portion, and the cutting trigger operably actuates a cutter actuation member in the end effector to perform a cutting action.

It is provided in the present invention an electrosurgical instrument, comprising: a handle assembly operably providing driving force and electrosurgical energy to an end effector, the handle assembly comprising a handle portion and a closure trigger pivotally connected to the handle portion, wherein the handle portion has a first accommodating chamber, the closure trigger has a second accommodating chamber, and an activation switch capable of turning on or off activation of transmission of the electrosurgical energy is provided in the second accommodating chamber; the area of the portion of the closure trigger received within the first accommodating chamber increases when the closure trigger is operated to pivot towards the handle portion, and the activation switch is triggered into an activated state when the closure trigger is operated to pivot to an activation position; the area of the portion of the closure trigger received within the first accommodating chamber decreases when the closure trigger is operated to pivot in a direction away from the handle portion, and the activation switch is trigger to an un-activated state when the closure trigger pivots to a non-activation position.

The technical solution of the present invention has the following technical effects over the prior art:
In the electrosurgical instrument provided by the present invention, a handle portion and/or a closure trigger has an accommodating chamber. An activation switch for closing or opening an activation circuit is located in the accommodating chamber. When the closure trigger pivots from an initial position towards the handle portion side to a closed position and then further pivots to an activation position, the activation switch is triggered to excite electrosurgical energy. The closure trigger as one same button is linked to the activation circuit, effectively shortening an operation time of closing and coagulating (cauterizing and sealing) tissue. In addition, the activation switch is located inside the handle portion and/or the closure trigger, thus effectively avoiding the problem of touching an activation button due to an operation error, and reducing safety risks to patients.

### BRIEF DESCRIPTION OF THE DRAWINGS

Preferred embodiments in the present invention will be described in detail below with reference to the accompanying drawings, thereby facilitating understanding of the objectives and advantages of the present invention, where:
FIG. 1 is a schematic structural diagram of a specific embodiment of an electrosurgical instrument according to the present invention;
FIG. 2 is a schematic diagram of an electrosurgical instrument having an electrical connection portion in the form of a plug according to the present invention;
FIG. 3 is a schematic diagram of an electrosurgical instrument having an electrical connection portion in the form of an electric slip ring according to the present invention;
FIG. 4 is a schematic diagram of a specific embodiment of a push-button switch employed in an electrosurgical instrument according to the present invention;
FIG. 5A is a schematic diagram of a closure trigger in a closed position in a specific embodiment of an electrosurgical instrument according to the present invention;
FIG. 5B is a schematic diagram of a closure trigger in an activation position in a specific embodiment of an electrosurgical instrument according to the present invention;
FIG. 5C is a schematic diagram of a closure trigger in an initial open position in a specific embodiment of an electrosurgical instrument according to the present invention;
FIG. 6 is a schematic structural diagram of the interior of a closure trigger in a first specific embodiment of an electrosurgical instrument employing a button switch according to the present invention;
FIG. 7 is a schematic structural diagram of a first trigger assembly in the first specific embodiment of an electrosurgical instrument employing a button switch according to the present invention;
FIG. 8 is a schematic structural diagram of a button switch and a control circuit board in the first specific embodiment of an electrosurgical instrument employing a button switch according to the present invention;
FIG. 9A is a schematic structural diagram of an initial open position in a second specific embodiment of an electrosurgical instrument employing a button switch according to the present invention;
FIG. 9B is a schematic structural diagram of an activation position in the second specific embodiment of an electrosurgical instrument employing a button switch according to the present invention;
FIG. 10A is a schematic structural diagram of an initial open position in a third specific embodiment of an electrosurgical instrument employing a button switch according to the present invention;
FIG. 10B is a schematic structural diagram of an activation position in the third specific embodiment of an electrosurgical instrument employing a button switch according to the present invention;
FIG. 11A is a schematic structural diagram of an initial open position in a fourth specific embodiment of an electrosurgical instrument employing a button switch according to the present invention;
FIG. 11B is a schematic structural diagram of an activation position in the fourth specific embodiment of an electrosurgical instrument employing a button switch according to the present invention;
FIG. 12A is a schematic structural diagram of an initial open position in a first specific embodiment of an electrosurgical instrument employing a contact switch according to the present invention;
FIG. 12B is a schematic structural diagram of an activation position in the first specific embodiment of an electrosurgical instrument employing a contact switch according to the present invention;
FIG. 13 is a schematic structural diagram of a pin contact in the first specific embodiment of an electrosurgical instrument employing a contact switch according to the present invention;
FIG. 14A is a schematic structural diagram of an initial open position in a second specific embodiment of an electrosurgical instrument employing a contact switch according to the present invention; and
FIG. 14B is a schematic structural diagram of the closure trigger side in the second specific embodiment of an electrosurgical instrument employing a contact switch according to the present invention.

### DETAILED DESCRIPTION

The technical solutions of the present invention will be described clearly and completely below with reference to the accompanying drawings. Evidently, the described embodiments are some of the embodiments of the present invention, rather than all of the embodiments. All other embodiments obtained by those of ordinary skill in the art on the basis of the embodiments of the present invention without the exercise of inventive effort shall fall within the scope of protection of the present invention.

In the description of the present invention, it should be noted that the orientation or positional relationship indicated by the terms "center", "upper", "lower", "left", "right", "vertical", "horizontal", "inner", "outer" and the like is based on the orientation or positional relationship shown in the accompanying drawings, and is only for convenience of description and simplicity of description, and does not indicate or imply that an indicated device or element must have a specific orientation and be constructed and operated in a specific orientation, and thus should not be construed as limiting the present invention. Furthermore, the terms "first", "second", and "third" are used for descriptive purposes only and are not to be construed as indicating or implying relative importance.

In the description of the present invention, it should be noted that, unless explicitly specified and defined otherwise, the terms "mounted", "connected", and "joined" are to be understood in a broad sense, for example, fixedly connected, detachably connected, integrally connected, directly joined, joined via an intermediate medium, or communication between the interiors of two components. The specific meaning of the above terms in the present invention will be understood by those of ordinary skill in the art in a specific context.

In addition, the technical features involved in various embodiments of the present invention described below can be combined with one another as long as they do not constitute a conflict therebetween.

In various embodiments of the present invention "distal end/side" refers to the end of a surgical instrument that is distal from an operator when operated, and "proximal end/side refers to the end/side of a surgical instrument that is proximal to an operator when operated.

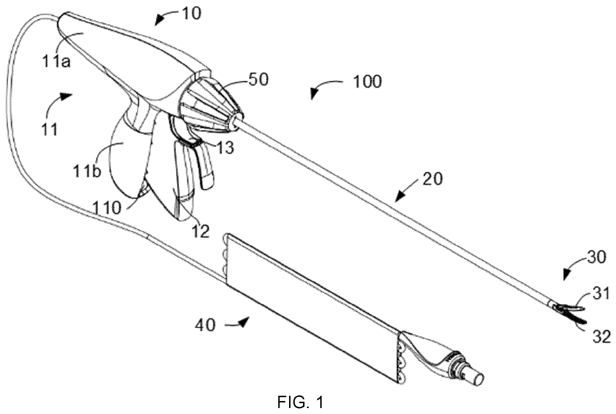

The present application relates generally to a surgical instrument, and particularly to an electrosurgical instrument, which is also referred to as a bipolar electrosurgical instrument and can be used to cut, coagulate (cauterize and seal) and/or grasping tissue during surgery, for example, during open surgery or during laparoscopic or endoscopic surgery. The surgical instrument operably transmits electrosurgical energy to an end effector 30 to act on tissue to thus coagulate (cauterize and seal) the tissue. As in FIG. 1, the surgical instrument described herein may also be used to grasp the tissue when not providing electrosurgical energy to the end effector 30. A jaw member of the end effector 30 may be selectively opened so that the end effector 30 holds the tissue to apply electrosurgical energy thereto.

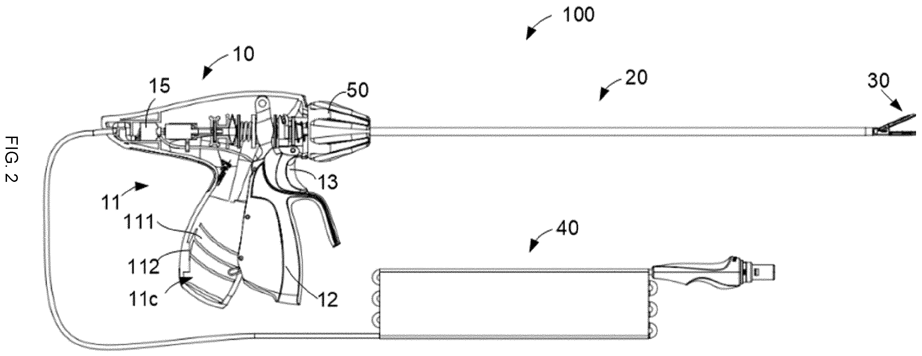

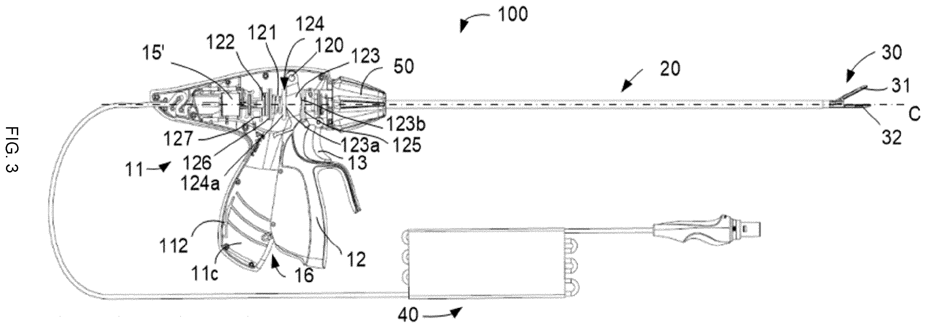

As shown in FIG. 1, an electrosurgical instrument 100 provided in the present invention is typically connected to an electrosurgical generator (not shown) that outputs electrosurgical energy. It can be understood that the electrosurgical instrument 100 may also be used with other various types of electrosurgical generators via a cable 40. The electrosurgical instrument 100 includes a handle assembly 10, an elongated body assembly 20, and the end effector 30 coupled in sequence from a proximal end to a distal end. An electrical connection portion 15 is provided in the proximal end portion of the handle assembly 10, adapted for being electrically connected to the electrosurgical generator. For example, in a specific embodiment shown in FIG. 2, the electrical connection portion 15 is designed as a socket or a plug, and is configured to be easily connected to an output terminal or an output connection socket of the electrosurgical generator. As an alternative embodiment, as shown in FIG. 3, an electrical connection portion 15' may also be configured in the form of an electric slip ring so as to provide a reliable electrical connection when components are rotated.

The end effector 30 is used to manipulate tissue to perform specific surgical procedures, such as tissue grasping, coagulating, and cutting. Referring to FIG. 1, the end effector 30 includes a first jaw 31 and a second jaw 32 which are pivotally connected to each other. The first jaw 31 and the second jaw 32 pivot in a direction close to each other to clamp tissue, and the first jaw 31 and the second jaw 32 pivot in a direction away from each other to release the tissue. Alternatively, in an alternative embodiment, the first jaw 31 of the end effector 30 operably pivots towards the second jaw 32 until the jaws of the end effector 30 are closed to clamp tissue, and the first jaw 31 pivots in a direction away from the second jaw 32 until the jaws of the end effector 30 are open to release the tissue, or vice versa. Further, the first jaw 31 and the second jaw 32 each include an electrode plate for contacting the tissue to transmit the electrosurgical energy to the tissue to perform electrocoagulation.

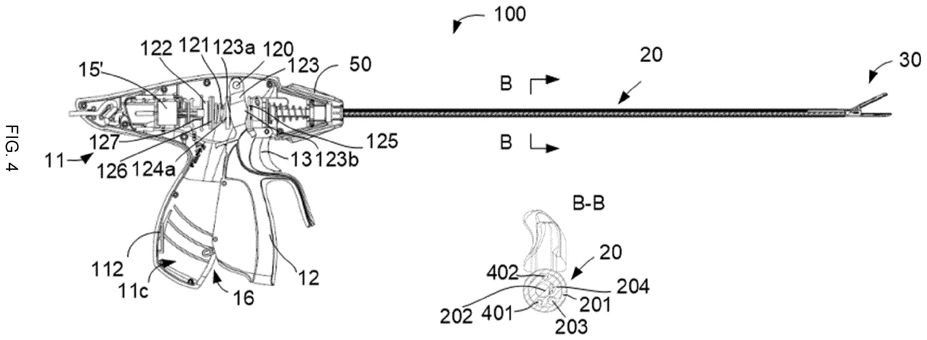

As further shown in FIGS. 1 to 3, at least a portion of the handle assembly 10 is to be held by a user to facilitate manipulation of the surgical instrument by the operator. The handle assembly 10 operably provides driving force, e.g., closing driving force and cutting driving force, to the end effector 30. The handle assembly 10 includes a handle portion 11 that can be gripped by the user and a closure trigger 12 pivotally connected to the handle portion 11. The user operates the closure trigger 12 to operate the end effector 30 to perform a closing or opening action. In a specific embodiment, the handle portion 11 includes a first half-housing and a second half-housing. The first half-housing and the second half-housing may be detachably connected by means of a snap connection, a fastener connection, etc. The handle portion 11 is substantially T-shaped, and includes a body portion 11a extending in a direction of a longitudinal axis C and a grip portion 11b extending in a direction substantially perpendicular to the longitudinal axis or inclined at a certain angle relative to the longitudinal axis. A first accommodating chamber 11c is formed inside the body portion 11a and the grip portion 11b, and can accommodate a driving mechanism and an activation circuit. The elongated body assembly 20 includes a plurality of longitudinal components that operably couple the end effector 30 to a plurality of actuators accommodated by the handle assembly 10. The elongated body assembly 20 includes an outer sheath 201. The outer sheath 201 defines the outer surface of the elongated body assembly, and allows other components to move and pass therethrough. For example, as specifically shown in FIG. 4, the outer sheath 201 is adapted to move longitudinally relative to an inner actuation member 202 that is axially received in the outer sheath 201. The inner actuation member 202 may be designed as a rod, a shaft, stamped metal, or other suitable metal component.

Referring to FIG. 4, the closure trigger 12 is operably coupled on a proximal portion of the elongated body assembly 20 via a connection portion 123. Specifically, the connection portion 123 is formed at an upper end portion of the closure trigger 12 and is engaged to the proximal portion of the outer sheath 201. The connection portion 123 is pivotably mounted in the housing of the handle portion 11 via a pivot 120. Specifically, the connection portion 123 extends upwards around opposite side portions of a driving collar 124 provided on the outer sheath 201, and includes a curved proximal driving surface 123a and a curved distal driving surface 123b thereon. The proximal driving surface 123a is engaged with a distal surface of a spring washer 124a of the driving collar 124. A snap ring 125 is further coupled on the outer sheath 201. The snap ring 125 is actuated to move with longitudinal movement of the outer sheath 201. The driving surface 123b is engaged with a proximal surface of the snap ring 125. Further, a proximal washer 126 is further coupled on the outer sheath 201. The proximal washer 126 is actuated to move with the longitudinal movement of the outer sheath 201. A first biasing member 121 is coupled on the outer sheath 201, and is arranged between a distal surface of the proximal washer 126 and the driving collar 124. A stopper 127 is provided on the housing of the handle portion 11. A second biasing member 122 is provided between a proximal surface of the proximal washer 126 and a distal surface of the stopper 127. In some specific embodiments, an elastic coefficient of the second biasing member 122 is lower than that of the first biasing member 121, so that when the first biasing member 121 and/or the second biasing member 122 are compressed, a different feel of grip is provided to the user.

Referring to FIG. 4, the handle assembly 10 further includes a cutting trigger 13 pivotally coupled to the handle portion 11, and the user operates the cutting trigger 13 to operate a cutter actuation member 203 in the end effector 30 to perform a cutting action. Cables 401 and 402 for transmitting electrosurgical energy are further provided in the outer sheath 201, and distal ends of the cables 401 and 402 are respectively connected to the first jaw 31 and the second jaw 32 of the end effector 30 so as to transmit high-frequency electrosurgical energy to the tissue coagulation and closing electrode pieces on the first jaw 31 and the second jaw 32. In some specific embodiments, the elongated body assembly 20 further includes an inner support tube 204 used to support and guide arrangement of the inner actuation member 202, the cutter actuation member 203, and the cables 401 and 402. In addition, a distal end portion of the handle assembly 10 is further provided with a knob 50. The user may operate and rotate the knob 50 to cause the elongated body assembly 20 and the end effector 30 to rotate as a whole about the longitudinal axis C. A side of the closure trigger 12 located away from the connection portion 123 extends to be opposite the body portion 11a of the handle portion 11. The grip portion 11b of the handle portion 11 has an opening 110 on a side opposite the closure trigger 12. The closure trigger 12 is slidable along the opening 110 to partially slide in or out of the first accommodating chamber 11c.

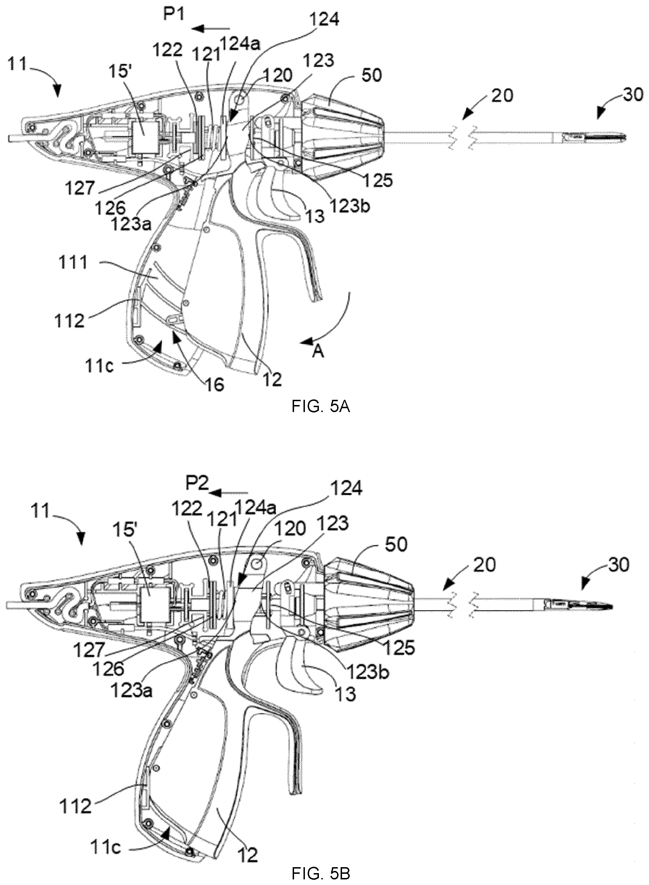

Next, a specific structure of the electrosurgical instrument 100 provided by the present invention for performing closing, activation, and opening operations will be described in detail with reference to the accompanying drawings. Specifically, as shown in FIG. 5A, when the closure trigger 12 is operated to pivot in a direction (shown as arrow A) toward the handle portion 11, the area of the closure trigger 12 received within the first accommodating chamber 11c gradually increases, and the proximal driving surface 123a of the connection portion 123 of the closure trigger 12 drives the spring washer 124a of the driving collar 124 to move proximally (shown as arrow P1). The elastic coefficient of the first biasing member 121 is greater than the elastic coefficient of the second biasing member 122, so that under the action of the first biasing member 121, the proximal washer 126 is also actuated to move proximally (arrow P1) accordingly, thereby further compressing the second biasing member 122. The proximal washer 126 is longitudinally fixedly connected to the outer sheath 201 of the elongated body assembly 20, so that the outer sheath 201 is operated to move proximally (arrow P1) accordingly, thereby achieving relative movement of the outer sheath 201 and the inner actuation member 202. An included angle formed between the first jaw 31 and the second jaw 32 of the end effector 30 gradually decreases until the closure trigger 12 is pivoted to a closed position, and the end effector 30 is in a closed state.

As shown in FIG. 5B, when the closure trigger 12 is further operated to pivot in a direction toward the handle portion 11, the area of the portion of the closure trigger 12 received within the first accommodating chamber 11c further increases. The proximal driving surface 123a of the connection portion 123 of the closure trigger 12 drives the spring washer 124a of the driving collar 124 to further move proximally (arrow P2), and the first biasing member 121 is further compressed. The spring washer 124a is longitudinally movable relative to the outer sheath 201 of the elongated body assembly 20, so that the outer sheath 201 does not move with the proximal movement of the spring washer 124a, and the end effector 30 remains in the closed state. When the closure trigger 12 is operated to pivot to an activation position, for example, as shown in FIG. 5B, a trigger assembly 16 is triggered to enable the activation circuit, and the electrosurgical energy is provided to the end effector 30. The elastic coefficient of the first biasing member 121 is greater than that of the second biasing member 122, so that when the closure trigger 12 is operated to pivot from the closed position to the activation position, the first biasing member 121 provides greater biasing force to the operator, thereby allowing the operator to distinguish between a closing operation and an activation operation according to the difference in the feel of grip.

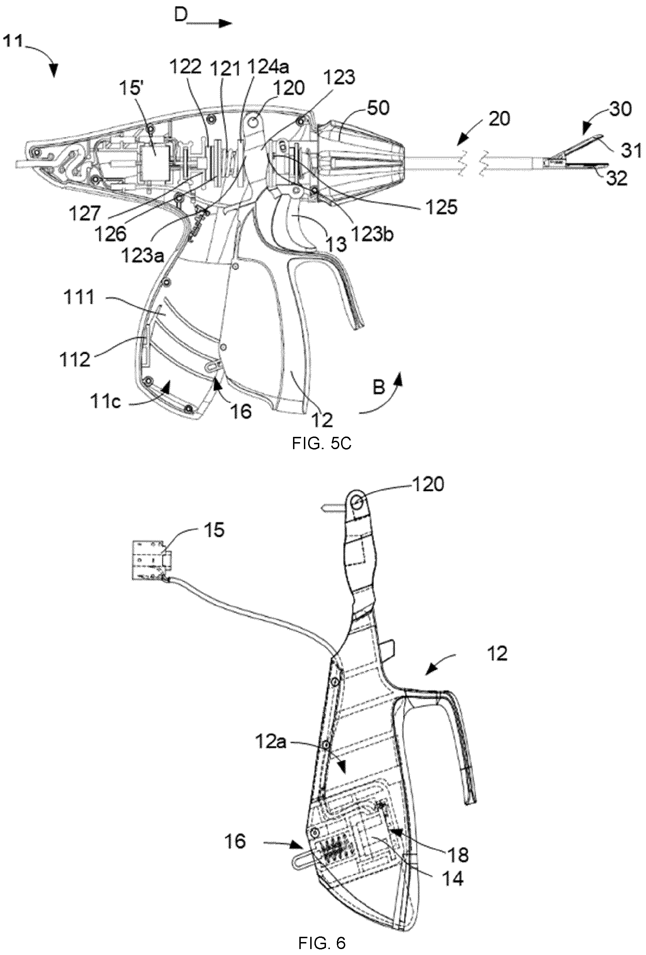

As further shown in FIG. 5C, after the activation operation is completed, when the closure trigger 12 is operated to pivot in a direction (arrow B) away from the handle portion 11, the area of the portion of the closure trigger 12 received within of the first accommodating chamber 11c decreases. Under the action of return force provided by the first biasing member 121 and/or the second biasing member 122, the distal driving surface 123b of the closure trigger 12 drives the snap ring 125 to move distally (arrow D), so that the outer sheath 201 of the elongated body assembly 20 moves distally relative to the inner actuation member 202. Therefore, the included angle formed between the first jaw 31 and the second jaw 32 of the end effector 30 increases. The end effector 30 gradually opens until the closure trigger 12 is operated to pivot to an open position, and the end effector 30 is in a maximally open state. In some specific embodiments, when the closure trigger 12 pivots to the open position, the closure trigger 12 is at least partially located in the first accommodating chamber 11c. Certainly, it can be understood that the user may also choose to switch between the closing operation and the opening operation to hold and open target tissue, rather than performing electrocoagulation.

As further shown in FIGS. 2 to 4, a guide structure is provided between an inner wall of the first accommodating chamber 11c of the handle portion 11 and an outer wall surface of the closure trigger 12 to support and guide pivoting movement of the closure trigger 12, so as to prevent the closure trigger 12 from shaking in the first accommodating chamber 11c. The guide structure includes a first guide protrusion 111 or a first guide recess provided on the inner wall of the first accommodating chamber 11c, and a second guide recess or a second guide protrusion provided on the outer wall surface of the closure trigger 12 and engaging therewith. For example, the first guide protrusion 111 on the inner wall of the first accommodating chamber 11c may engage with the second guide recess (not shown) on the outer wall surface of the closure trigger 12 to directionally guide a closing or opening action of the closure trigger 12. Certainly, the first guide protrusion 111 on the inner wall of the first accommodating chamber 11c may engage with the second guide protrusion (not shown) on the outer wall surface of the closure trigger 12 to directionally guide relative sliding therebetween. In a specific embodiment, the closure trigger 12 includes a second accommodating chamber 12a. The second accommodating chamber 12a is an open chamber open towards the handle portion 11. An activation switch is provided in the first accommodating chamber 11c and/or the second accommodating chamber 12a, and is configured to operably turn on or off the electrosurgical energy for the electrosurgical instrument 100. For example, when the closure trigger 12 pivots to the activation position, the activation switch 14 is triggered to cause the activation circuit to switch to a closed state, and the electrosurgical energy outputted by the electrosurgical generator is outputted via the electrical connection portion 15 to the end effector 30, so as to apply the electrosurgical energy to tissue on the end effector 30 to coagulate the tissue, thereby achieving closing and activation operations by the closure trigger 12 as one same button, and shortening an operation time of using the instrument to hold and coagulate the tissue. In addition, the activation switch is located in the handle portion 11 and/or the closure trigger 12, thus effectively avoiding the problem of touching an activation button due to an operation error, and reducing safety risks to patients.

In an alternative embodiment, the closure trigger 12 has a solid end surface facing the handle portion 11, and the solid end surface is not provided with an open chamber. The activation switch 14 is provided in the first accommodating chamber 11c of the handle portion 11. An end portion of the closure trigger 12 is provided with a trigger structure for triggering the activation switch. The closure trigger 12 pivots towards the inside of the first accommodating chamber 11c of the handle portion 11 to the activation position, so as to trigger the activation switch 14 into the on state. Corresponding to the embodiment, the handle portion 11 has a solid end surface facing the closure trigger 12, and the solid end surface is not provided with an open chamber. The activation switch is provided in the second accommodating chamber 12a of the closure trigger 12. An end portion of the handle portion 11 is provided with a trigger structure for triggering the activation switch.

Specifically, at least a portion of the activation circuit is arranged on a control circuit board 18, and the activation switch is located on a connection cable between the electrical connection portion 15 and the control circuit board 18. In an alternative embodiment, the control circuit board 18 is mounted in the second accommodating chamber 12a of the closure trigger 12, and when the closure trigger 12 is operated to pivot to the open position, the closure trigger 12 is still partially located in the first accommodating chamber 11c, so that the control circuit board 18 is located in the second accommodating chamber 12a so as to protect the control circuit board 18, thereby preventing external dust or liquid from directly contacting the control circuit board 18 along a gap between the handle portion 11 and the closure trigger 12. In another embodiment, the control circuit board 18 may also be mounted in the first accommodating chamber 11c of the handle portion 11.

The activation switch 14 may be a button switch. The circuit is turned on by pressing a button of the button switch, and the circuit is turned off by releasing the button of the button switch. Alternatively, in another embodiment, the activation switch 14 may also be a contact switch, and two contacts thereof contact each other or separate to cause the circuit to be turned on or off. It can be understood that the activation switch 14 may also be in the form of another switch capable of causing the circuit to be on or off. For example, in some specific embodiments in which the activation switch 14 is a button switch, the trigger assembly 16 for triggering the button switch is further included in the first accommodating chamber 11c and/or the second accommodating chamber 12a. When the closure trigger 12 pivots to a first position, the trigger assembly deviates from an initial state and acts on the button switch to cause to the activation circuit to be in the closed state.

FIG. 2 to FIG. 8 show an embodiment of the electrosurgical instrument 100 provided in the present invention. In the embodiment, the activation switch 14 is designed as a button switch. The trigger assembly 16 for triggering the activation switch 14 is further provided in the second accommodating chamber 12a of the closure trigger 12, and includes a trigger lever 161 slidably connected to a mounting seat 19, and a biasing member 162 mounted on the mounting seat 19 and acting on the trigger lever 161. The trigger lever 161 slides on the mounting seat 19 in a first direction to trigger the activation switch 14 to be turned on or off. The activation switch 14 is provided in the second accommodating chamber 12a, and an contact portion 112 for engaging with the trigger assembly 16 is provided in the first accommodating chamber 11c. When the closure trigger 12 is in a non-activation position (e.g., the closed position, the open position, or a certain position of an intermediate state), the trigger lever 161 under the action of biasing force provided by the biasing member 162 is in an initial position, and in this case, the trigger lever 161 is kept at a preset distance from the activation switch 14. When the closure trigger 12 pivots to the activation position, a first end 161a (a proximal end) of the trigger lever 161 abuts the contact portion 112, and a second end 161b (a distal end) of the trigger lever 161 overcomes elastic force of the biasing member 162 to act on the activation switch 14, so that the activation switch 14 is in a pressed state, and the activation circuit is turned on. When the closure trigger 12 pivots away from the handle portion 11, the trigger lever 161 moves along with the closure trigger 12 and away from the contact portion 112, and the trigger lever 161 under the action of the biasing member 162 slides again in a direction away from the activation switch 14 to the initial position.

In an alternative embodiment, one end of the biasing member 162 abuts the trigger lever 161, and the other end of the biasing member 162 directly abuts the activation switch 14, so that the activation switch 14 is maintained in an activated state during at least part of movement of the closure trigger 12 from an actuated position of the activation switch 14 to an unactuated position of the activation switch 14. Specifically, pressing of the trigger lever 161 causes the biasing member 162 to be compressed between the trigger lever 161 and the activation switch 14, and causes biasing force to be applied to the activation switch 14. If, during operation of the cutting trigger 13, the closure trigger 12 is subjected to inadvertent operation to move distally by a certain angle, for example, a pivoting angle of the closure trigger 12 is smaller than, for example, 5°, the biasing member 162 (i.e., currently slightly decompressed) compensates for or partially counteracts the effect of the distal movement of the closure trigger 12 via spring force maintained on the activation switch 14. During movement of the closure trigger 12 in a distal direction, the spring force maintained on the activation switch 14 by the biasing member 162 is sufficient to keep the same in the on state, so that the activation switch 14 is not released and turned on again during operation of the cutting trigger 13. In the process of operating the closure trigger 12 to move away from the contact portion 112 to cause the end effector 30 to the open state, acting force for triggering the trigger lever 161 to move is less than acting force for triggering the activation switch 14, so that the biasing member 162 moves along with the trigger lever 161 to the initial position, and the spring force on the activation switch 14 decreases or eliminates , thereby releasing the activation switch 14.

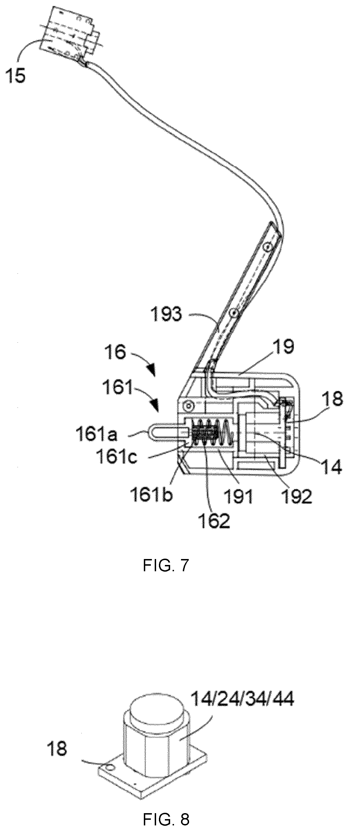

As shown in FIG. 2 to FIG. 4, the contact portion 112 in the first accommodating chamber 11c is formed as an inner wall surface of the first accommodating chamber 11c. More specifically, the inner wall surface protrudes relative to other regions of the first accommodating chamber 11c, and a surface engaging with the trigger lever 161 is a flat surface. As shown in FIGS. 5 to 7, the trigger lever 161 is formed so that an end portion of the first end 161a has a curved surface so as to smoothly contact the contact portion 112. The second end 161b of the trigger lever 161 is formed in the form of a cylindrical rod, and is in surface contact with a pressing surface of the activation switch 14. A sliding portion 161c is further provided between the first end 161a and the second end 161b of the trigger lever 161. As optimally shown in FIG. 7, the mounting seat 19 is provided with a first mounting groove 191 extending in the first direction. The sliding portion 161c of the trigger lever 161 engages with a groove wall of the first mounting groove 191 to achieve sliding connection. Two opposite side surfaces of the first mounting groove 191 are provided with a first through hole and a second through hole. The first end 161a and the second end 161b of the trigger lever 161 may respectively extend out of the first through hole and the second through hole to the outside of the first mounting groove 191. In the embodiment, the biasing member 162 is a compression spring. The compression spring is sleeved on the second end 161b of the trigger lever 161, and has one end abutting the sliding portion 161c.

In the initial state or an un-activated state, the compression spring causes the sliding portion 161c of the trigger lever 161 to abut a first side surface (a proximal side surface) of the first mounting groove 191, so that at least a portion of the first end 161a of the trigger lever 161 is located outside the first mounting groove 191. After the closure trigger 12 is operated to move in a direction of approaching the handle portion 11 to cause the first end 161a of the trigger lever 161 of the trigger assembly 16 to abut the contact portion 112 of the handle portion 11, the trigger lever 161 is pushed to move in a direction away from the contact portion 112, so that an end portion of the second end 161b of the trigger lever 161 is located outside the first mounting groove 191, and acts on a button switch 14a located outside the first mounting groove 191.

In an optional embodiment, as shown in FIG. 6 and FIG. 7, the activation switch 14 and the control circuit board 18 are both mounted on the mounting seat 19, so as to be mounted in an integrated manner. The mounting seat 19 is further provided with a second mounting groove 192 adjacent to the first mounting groove 191. The activation switch 14 engages in the second mounting groove 192, and a pressing portion of the activation switch 14 faces the second through hole of the first mounting groove 191 so as to be pressed and triggered by the second end 161b of the trigger lever 161.

As shown in FIG. 7, the mounting seat 19 is further provided with a cable mounting portion 193 for fixing a cable. The cable mounting portion 193 is formed as a rod extending in a second direction, and the rod is provided with several clips for fixing the cable via binding. The rod is provided near a distal inner wall of the closure trigger 12. The mounting seat 19 is fixed to an inner wall of the second accommodating chamber 12a of the closure trigger 12 by means of engagement or insertion. Specifically, the inner wall of the second accommodating chamber 12a of the closure trigger 12 has several slots formed by ribs. An outer wall of the mounting seat 19 is inserted in the slot. A positioning protrusion and a positioning recess are provided between the slot and the mounting seat 19, and reliable mounting and fixation therebetween are achieved via the deformable positioning structure of the mounting seat 19.

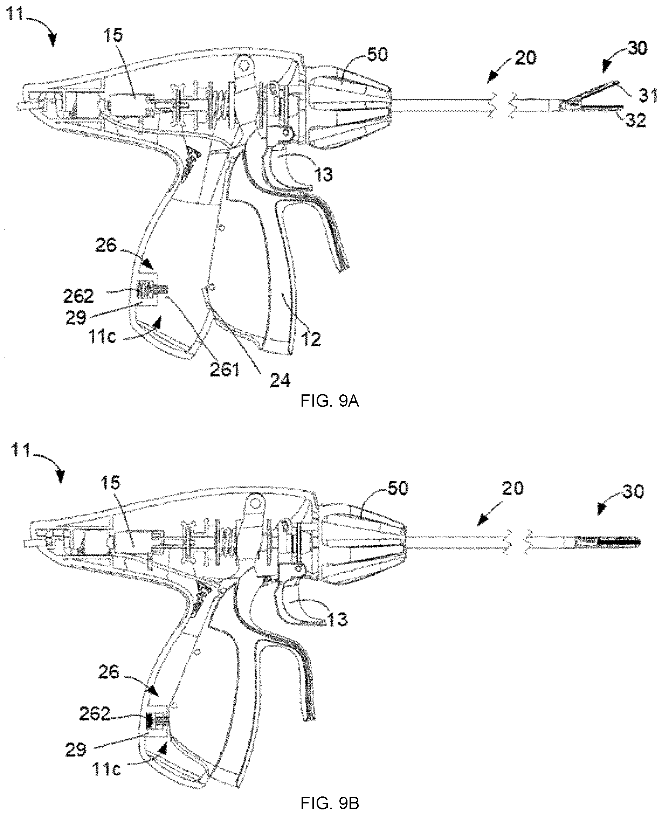

FIGS. 9A and 9B show another embodiment of the electrosurgical instrument 100 provided in the present invention. In the embodiment, an activation switch 24 is configured to be a button switch, and is at least partially provided in the second accommodating chamber 12a of the closure trigger 12. A trigger assembly 26 is located in the first accommodating chamber 11c, and includes: a trigger lever 261 slidably connected to a mounting seat 29 and a biasing member 262 mounted on the mounting seat 29 and acting on the trigger lever 261. The trigger lever 261 slides on the mounting seat 29 in a second direction to trigger the activation switch 24 to be turned on or off. The second direction may be the same as or different from the first direction. At least a portion of the activation switch 24 is located in the second accommodating chamber 12a. When the closure trigger 12 is in a non-activation position, the trigger lever 261 under the action of the biasing member 262 extends towards an end portion of the activation switch 24 and out of the mounting seat 29. When the closure trigger 12 pivots to the activation position, the trigger lever 261 overcomes elastic force of the biasing member 262, and acts on the activation switch 24, so that the activation switch 24 is in a pressed state, and the activation circuit is closed. When the closure trigger 12 pivots away from the handle portion 11, the activation switch 24 moves accordingly and away from the trigger lever 261. The activation switch 24 is triggered to an un-activated state, and the activation circuit is turned off. The trigger lever 261 under the action of the biasing member 262 slides again to the initial position.

The mounting seat 29 is integrally formed on an inner wall surface of the handle portion 11. The mounting seat 29 has a mounting groove 291 extending in the second direction. A side of the mounting seat 29 facing the activation switch 24 is provided with an opening. The trigger lever 261 includes a sliding portion 261c engaging with the mounting groove 291, a distal lever portion 261a extending distally from the sliding portion 261c and running through the opening, and a proximal lever portion 261b extending proximally from the sliding portion 261c. The biasing member 262 may be a compression spring, is sleeved on the proximal lever portion 261b, has one end abutting the sliding portion 261c, and has the other end abutting an inner wall of the mounting seat 29.

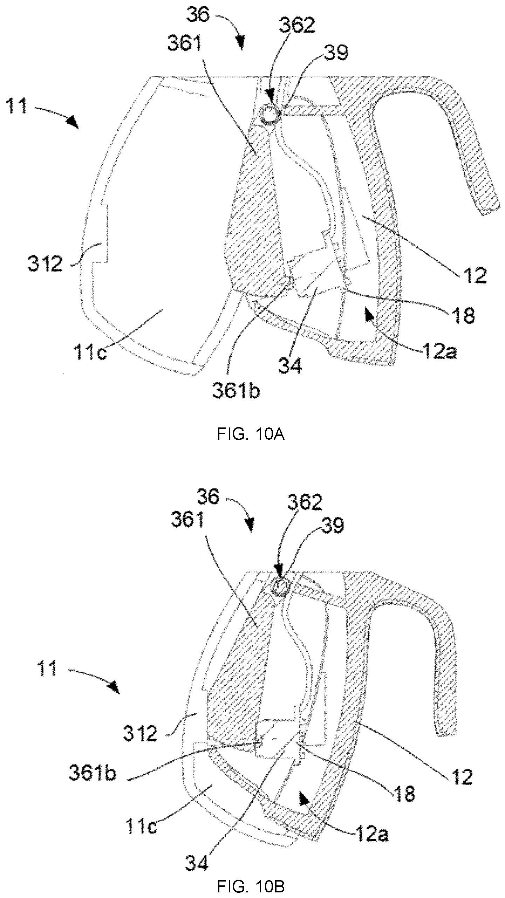

FIGS. 10A and 10B show another embodiment of the electrosurgical instrument 100 provided in the present invention. In the embodiment, an activation switch 34 is a button switch, and a trigger assembly 36 is at least partially provided in the second accommodating chamber 12a of the closure trigger 12. Specifically, the trigger assembly 36 includes: a trigger lever 361 pivotably connected to a mounting seat 39 and a biasing member 362 (not shown) mounted on the mounting seat 39 and acting on the trigger lever 361. The trigger lever 361 swings in a third direction to trigger the activation switch 34 to be turned on or off. The activation switch 34 is located in the second accommodating chamber 12a of the closure trigger 12. A contact portion 312 for engaging with the trigger assembly 16 is provided in the first accommodating chamber 11c of the handle portion 11. When the closure trigger 12 is in a non-activation position, the trigger lever 361 under the action of the biasing member 362 is in an initial position in which the trigger lever abuts the activation switch 34 but does not trigger the activation switch 34 or there is a set gap. When the closure trigger 12 pivots to the activation position, a first end (a proximal end) 361a of the trigger lever 361 abuts the contact portion 312, and a second end (a distal end) 361b of the trigger lever 361 overcomes biasing force of the biasing member 362 and acts on the activation switch 34, so that the activation switch 34 is in the pressed state, and the activation circuit is closed. When the closure trigger 12 pivots away from the handle portion 11, the trigger lever 361 moves along with the closure trigger 12 and away from the contact portion 312. The trigger lever 361 under the action of the biasing member 362 slides again in a direction away from the activation switch 34 to the initial position or a non-activation position. The activation switch 34 is triggered to the un-activated state, and the activation circuit is off.

The contact portion 312 in the first accommodating chamber 11c is formed as an inner wall surface of the first accommodating chamber 11c. More specifically, the inner wall surface protrudes relative to other regions of the first accommodating chamber 11c, and a surface engaging with the trigger lever 361 is a flat surface. A pivot shaft or a pivot shaft sleeve is provided in the second accommodating chamber 12a to form the mounting seat 39, and a pivot shaft sleeve or a pivot shaft is correspondingly provided on the trigger lever 361, so as to achieve pivotal connection with the closure trigger 12. The biasing member 362 is a torsional spring sleeved on the pivot shaft. A side of the trigger lever 361 away from the mounting seat 39 extends to a position opposite a pressing portion of the activation switch 34, and the trigger lever 361 is provided with a trigger protrusion 361b on an end surface opposite the activation switch 34. The trigger protrusion 361b is used for acting on the activation switch 34 when the trigger lever 361 engages with the contact portion 312, so as to cause the same to be in the pressed state.

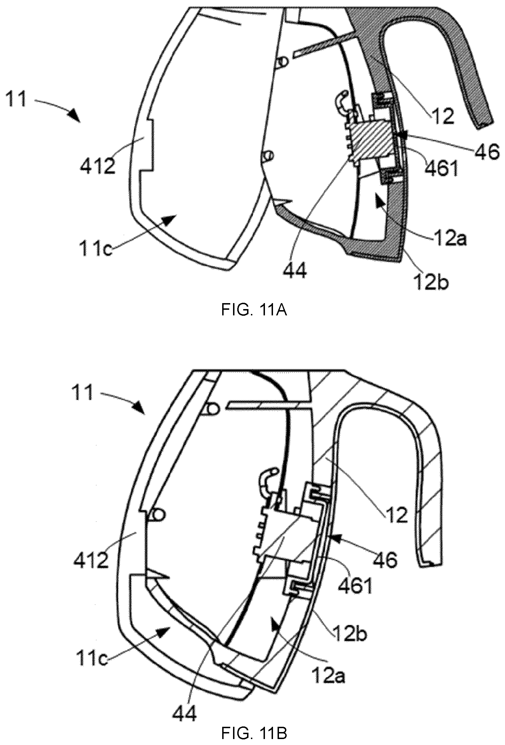

FIGS. 11A and 11B show another embodiment of the electrosurgical instrument 100 provided in the present invention. In the embodiment, an activation switch 44 is configured to be a button switch. In the specific embodiment, a housing surface of the closure trigger 12 is provided with a trigger assembly 46 for triggering the activation switch 44. When the closure trigger 12 pivots to the activation position, the trigger assembly 46 may be operated by a user to trigger the activation switch 44 into the on state.

Specifically, a surface of the housing of the closure trigger 12 away from the handle portion 11 is provided with a mounting port. A pressing side of the activation switch 44 faces the mounting port. The trigger assembly 46 includes a sealing cover body 461 mounted on the mounting port. The sealing cover body 461 has an elastically deformable region opposite the activation switch 44. The elastically deformable region of the sealing cover body 461 is capable of being triggered and deforming in a direction close to the activation switch 44 to a set value, to trigger the activation switch 44 to perform state switching.

In order to prevent the user from inadvertently triggering the trigger assembly 46 when the closure trigger 12 is in a non-activation position, triggering force of the elastically deformable region of the sealing cover body 461 is greater than closing pivoting force of the closure trigger 12. That is, when the closure trigger 12 is in the initial position, even if the user triggers the elastically deformable region of the sealing cover body 461, the closure trigger 12 is first driven to pivot in a direction close to the first position until the closure trigger 12 pivots to the activation position. The activation switch 44 abuts the contact portion 412, so that only in this case, the user can further cause the elastically deformable region of the sealing cover body 461 to deform to the set value, to trigger the activation switch 44 into the activated state.

Specifically, the sealing cover body 461 is connected to the mounting port side of the closure trigger 12 by means of engagement. A region gripped by the user on an outer wall of the closure trigger 12 is encapsulated, and an encapsulating layer 12b covers a position of engagement between the sealing cover body 461 and the mounting port, thereby preventing external dust or the like from entering the closure trigger 12 along a gap therebetween.

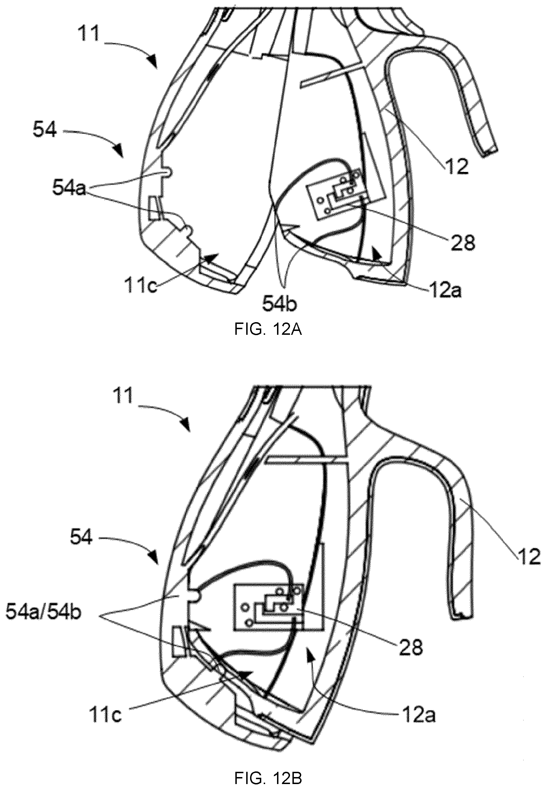

FIGS. 12A and 12B show another embodiment of the electrosurgical instrument 100 provided in the present invention. In the embodiment, an activation switch 54 is designed as a contact switch, and at least a portion of the activation circuit is arranged on a control circuit board 28. The activation switch 54 includes: at least one first contact 54a located in the first accommodating chamber 11c and at least one second contact 54b located in the second accommodating chamber 12a. The first contact 54a is electrically connected to the electrical connection portion 15. The second contact 54b is electrically connected to the control circuit board 28. When the closure trigger 12 is in a non-activation position, the first contact 54a and the second contact 54b are in a separated state, and the activation circuit is turned off. When the closure trigger 12 is operated to pivot to the activation position, the second contact 54b abuts the first contact 54a, turning on the activation circuit.

Specifically, two first contacts 54a connected in parallel to each other are provided in the first accommodating chamber 11c of the handle portion 11, and correspondingly, two second contacts 54b connected in parallel to each other are provided in the second accommodating chamber 12a of the closure trigger 12. When the closure trigger 12 is operated to pivot to the activation position, the two sets of the second contacts 54b and the first contacts 54a connected in parallel to each other engage with each other to turn on the activation circuit, thereby improving reliability of the function of the activation switch 14.

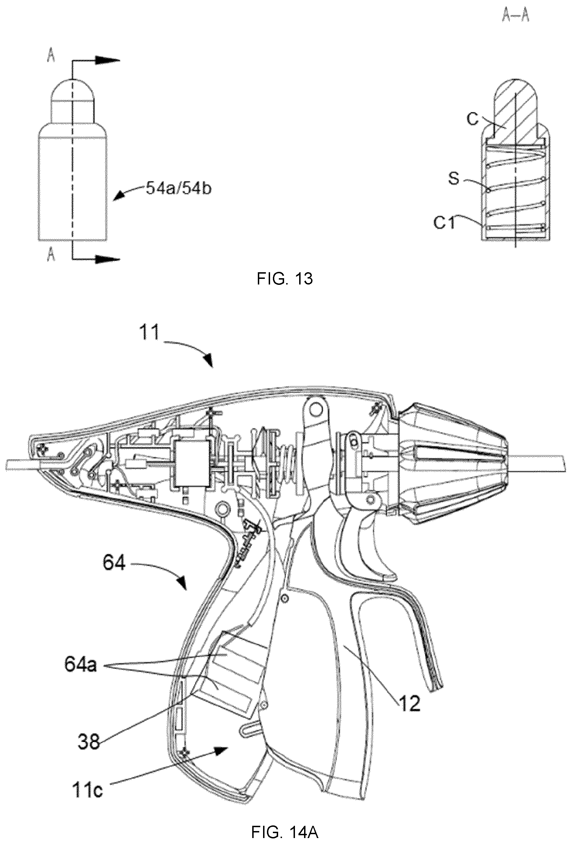

Further, the first contact 54a and the second contact 54b may be pin contacts. Specifically, as shown in FIG. 13, the pin contact includes a fixed base C1 and a movable pin C slidably connected to the fixed base C1. A compression spring S is provided between the movable pin C and the fixed base C1, and can ensure reliable conduction in a certain range when the first contact 54a and the second contact 54b contact each other.

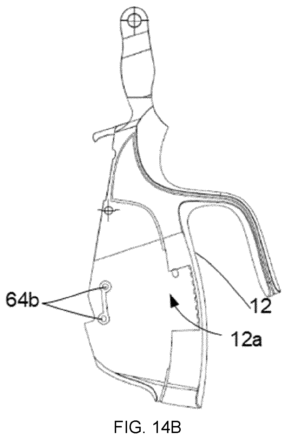

FIGS. 14A and 14B show another embodiment of the electrosurgical instrument 100 provided in the present invention. In the embodiment, an activation switch 64 is designed as a contact switch, and at least a portion of the activation circuit is arranged on a control circuit board 38. The activation switch 64 includes: at least one first contact 64a located in the first accommodating chamber 11c and at least one second contact 54b located in the second accommodating chamber 12a. The first contact 64a is electrically connected to the electrical connection portion 15 by means of the control circuit board 38. When the closure trigger 12 is in a non-activation position, the first contact 64a and the second contact 64b are in a separated state, and the activation circuit is turned off. When the closure trigger 12 is operated to pivot to the activation position, the second contact 64b abuts the first contact 64a, turning on the activation circuit.

Evidently, the above embodiments are merely examples for clear description, and are not intended to limit the implementations. Those of ordinary skill in the art could further make variations or modifications in other different forms in light of the above description. It is not necessary and possible to enumerate all embodiments exhaustively herein. Obvious variations and modifications derived therefrom also fall within the scope of protection of the present invention.

## Claims

1. An electrosurgical instrument, comprising:
an end effector; and
a handle assembly operably providing driving force and electrosurgical energy to the end effector, the handle assembly comprising a handle portion and a closure trigger pivotally coupled to the handle portion, **characterized in that**
a first accommodating chamber is arranged in the handle portion, a second accommodating chamber is arranged in the closure trigger, and an activation switch is arranged in the first accommodating chamber and/or the second accommodating chamber for enabling or disabling delivery of the electrosurgical energy, and
when the closure trigger is opertated to pivot toward the handle portion to an activation position, the activation switch is triggered into an activated state allowing the electrosurgical energy delivered to the end effector.

2. The electrosurgical instrument according to claim 1, wherein when the end effector is in an open state, at least a portion of the closure trigger is received in the first accommodating chamber of the handle portion.

3. The electrosurgical instrument according to claim 1, wherein when the closure trigger is pivoted toward the handle portion, the area of the portion of the closure trigger received within the first accommodating chamber increases; and as the closure trigger is pivoted away from the handle portion, the area of the portion of the closure trigger received within the first accommodating chamber decreases.

4. The electrosurgical instrument according to claim 1, further comprising an electrical connection portion for being electrically connected to an electrosurgical generator for generating the electrosurgical energy, and a control circuit board electrically connected to the electrical connection portion; wherein an activation circuit is arranged on the control circuit board, and the activation switch is operably configured to enable or disable the activation circuit to control delivery of the electrosurgical energy to the end effector.

5. The electrosurgical instrument according to claim 1, wherein the activation switch is a button switch, and a trigger assembly for triggering the activation switch is further provided in the first accommodating chamber and/or the second accommodating chamber, and when the closure trigger is pivoted to the activation position, the trigger assembly is biased from an initial state and acts on the activation switch to enable the activation switch.

6. The electrosurgical instrument according to claim 1, wherein the activation switch is provided in the second accommodating chamber, and when the closure trigger is pivoted to the activation position, the activation switch is operably triggered into the activated state.

7. The electrosurgical instrument according to claim 6, wherein the second accommodating chamber further comprises a trigger assembly having a trigger lever operable to be moved from an initial state so as to enable the activation switch, and the trigger lever is biased in the initial state through a biasing member engaged therewith.

8. The electrosurgical instrument according to claim 7, wherein a mounting seat is provided in the second accommodating chamber, the trigger lever of the trigger assembly is slidably provided on the mounting seat, and the activation switch is operated to be turned on or off through sliding of the trigger lever on the mounting seat in a first direction, and the biasing member is provided on the mounting seat to provide the biasing force to the trigger lever.

9. The electrosurgical instrument according to claim 8, wherein one end of the biasing member acts on the activation switch, the other end acts on the trigger lever of the trigger assembly, and the activation switch is maintained in the activated state during at least a portion of movement of the closure trigger pivoting from the activation position towards an open position.

10. The electrosurgical instrument according to claim 7, wherein a mounting seat is provided in the second accommodating chamber, the trigger lever of the trigger assembly is pivotally provided on the mounting seat, and the activation switch is operated to be turned on or off as the trigger lever pivots on the mounting seat in a third direction, and the biasing member is provided on the mounting seat to provide the biasing force to the trigger lever.

11. The electrosurgical instrument according to any one of claims 6 to 10, wherein a contact portion for being engaged with the trigger assembly is provided in the first accommodating chamber, and when the closure trigger is pivoted to the activation position, the contact portion abuts one end of the trigger lever, so that the trigger lever overcomes biasing force of the biasing member and enables the activation switch.

12. The electrosurgical instrument according to any one of claims 6 to 10, wherein the activation switch and a control circuit board are mounted on the mounting seat.

13. The electrosurgical instrument according to claim 6, wherein a trigger assembly is further provided in the second accommodating chamber, and is provided on a housing surface of the closure trigger for a user to hold, and when the closure trigger pivots to the activation position, the trigger assembly operably enables the activation switch.

14. The electrosurgical instrument according to claim 13, wherein the closure trigger has a mounting port, a pressing side of the activation switch faces the mounting port, the trigger assembly comprises a sealing cover body mounted on the mounting port, the sealing cover body has an elastically deformable region opposite the activation switch, and the elastically deformable region of the sealing cover body is capable of being triggered and deforming in a direction close to the activation switch to a set position to turn on the activation switch.

15. The electrosurgical instrument according to claim 14, wherein force for triggering pivoting the closure trigger is less than elastic deformation force for the sealing cover body.

16. The electrosurgical instrument according to claim 6, wherein a trigger assembly is further provided in the first accommodating chamber, and the trigger assembly comprises a trigger lever slidably connected to a mounting seat, the trigger lever sliding on the mounting seat in a second direction to trigger the activation switch to be turned on or off; and a biasing member mounted on the mounting seat and acting on the trigger lever.

17. The electrosurgical instrument according to claim 4, wherein the activation switch is a contact switch, and the activation switch comprises:
at least one first contact located in the first accommodating chamber and at least one second contact located in the second accommodating chamber, the first contact being electrically connected to the electrical connection portion, the first contact or the second contact being electrically connected to the control circuit board, and
when the closure trigger is operated to pivot to the activation position, the second contact abutting the first contact to enable the activation circuit.

18. The electrosurgical instrument according to claim 1, wherein a guide structure is provided between the handle portion and the closure trigger, and the guide structure is used for guiding a swinging direction of the closure trigger.

19. The electrosurgical instrument according to claim 1, wherein the handle assembly further comprises a first biasing member and a second biasing member, and the closure trigger is operated to pivot toward the handle portion to a closed position by overcoming elastic force of the second biasing member, and further to pivot toward the handle portion to the activation position by overcoming elastic force of the first biasing member and the second biasing member.

20. The electrosurgical instrument according to claim 19, further comprising an elongated body assembly defining a longitudinal axis, and comprising an outer sheath, and a proximal end of the outer sheath extending to the inside of the handle portion.

21. The electrosurgical instrument according to claim 20, wherein the closure trigger is operably coupled on a proximal portion of the outer sheath via a connection portion formed on an upper end portion of the closure trigger and pivotally mounted on the handle portion.

22. The electrosurgical instrument according to claim 21, wherein the handle portion is provided with a stopper located at a proximal portion of the outer sheath, a proximal washer, a driving collar, and a snap ring are sequentially sleeved on the outer sheath located in the handle portion from the proximal end to a distal end, the snap ring and the proximal washer are separately fixedly connected to the outer sheath, the driving collar is slidable along the outer sheath, a proximal end of the driving collar is provided with a spring washer, the connection portion of the closure trigger is sleeved on the driving collar, two ends of the connection portion respectively abut the spring washer and the snap ring, the first biasing member is provided between the spring washer and the proximal washer, the second biasing member is provided between the proximal washer and the stopper, and an elastic coefficient of the first biasing member is greater than an elastic coefficient of the second biasing member.

23. The electrosurgical instrument according to claim 1, wherein the handle assembly further comprises a cutting trigger pivotally connected to the handle portion, and the cutting trigger operably actuates a cutter actuation member in the end effector to perform a cutting action.

24. An electrosurgical instrument, comprising: a handle assembly operably providing driving force and electrosurgical energy to an end effector, the handle assembly comprising a handle portion and a closure trigger pivotally connected to the handle portion, **characterized in that**
the handle portion has a first accommodating chamber, the closure trigger has a second accommodating chamber, and an activation switch capable of turning on or off activation of transmission of the electrosurgical energy is provided in the second accommodating chamber; the area of the portion of the closure trigger received within the first accommodating chamber increases when the closure trigger is operated to pivot towards the handle portion, and the activation switch is triggered into an activated state when the closure trigger is operated to pivot to an activation position; the area of the portion of the closure trigger received within the first accommodating chamber decreases when the closure trigger is operated to pivot in a direction away from the handle portion, and the activation switch is trigger to an un-activated state when the closure trigger pivots to a non-activation position.
